(19)

Europäisches
Patentamt
European
Patent Office
Office européen
des brevets

(11) **EP 2 187 328 A1**

(12) **EUROPEAN PATENT APPLICATION**

(43) Date of publication:
**19.05.2010 Bulletin 2010/20**

(51) Int Cl.:
***G06F 19/00*** *(2006.01)*

(21) Application number: **08169327.7**

(22) Date of filing: **18.11.2008**

| | |
|---|---|
| (84) Designated Contracting States:<br>**AT BE BG CH CY CZ DE DK EE ES FI FR GB GR HR HU IE IS IT LI LT LU LV MC MT NL NO PL PT RO SE SI SK TR**<br>Designated Extension States:<br>**AL BA MK RS** | (72) Inventor: **The designation of the inventor has not yet been filed** |
| | (74) Representative: **Jungen, Carmen**<br>**Philips Intellectual Property &**<br>**Standards GmbH**<br>**Weißhausstraße 2**<br>**52066 Aachen (DE)** |
| (71) Applicant: **Koninklijke Philips Electronics N.V.**<br>**5621 BA Eindhoven (NL)** | |

(54) **Method and device for efficient searching of DNA sequence based on energy bands of DNA spectrogram**

(57)     The present invention discloses a method for DNA sequence analysis based on DNA spectrogram database. Furthermore, a use, a device and a computer-readable medium related to the method are disclosed.

EP 2 187 328 A1

**Description**

FIELD OF THE INVENTION

This invention pertains in general to the field of DNA sequences analysis. More particularly the invention relates to a method for DNA sequence analysis and a device for DNA sequence analysis.

BACKGROUND OF THE INVENTION

Bioinformatics seeks to organize tremendous volumes of biological data into comprehensible information, which can be used to derive useful knowledge.

One tool commonly used within the field of bioinformatics is the Basic Local Alignment Search Tool (BLAST). To run, BLAST requires a query sequence - also called the target sequence - to search for, and a sequence, or a sequence database containing multiple such sequences, to search against. Based on the query sequence, BLAST will find subsequences in the database which are similar to subsequences in the query. In typical usage, the query sequence is much smaller than the database, e.g., the query may be one thousand nucleotides while the database is several billion nucleotides.

A common problem for BLAST and other search tools known in the art is that the query sequence is limited. If the query sequence length is larger than around a few thousand nucleotides, the search tool will be unacceptably time consuming. Furthermore, with too large query sequences, the accuracy of the search tools diminishes.

In order to make existing bioinformatics tools faster and more accurate, the query sequence is usually manually modified and only the data that is deemed to be most relevant is used for searching. This subjective approch is leading to unreliable results because of unacceptable approximations.

DNA spectral analysis offers an approach to systematically tackle the problem of deriving useful information from DNA sequence data. Generally, DNA spectral analysis involves an identification of the occurrences of each nucleotide base in a DNA sequence as an individual digital signal, and transforming each of the four different nucleotide signals into a frequency domain. The magnitude of a frequency component can then be used to reveal how strongly a nucleotide base pattern is repeated at that frequency. A larger magnitude/value usually indicates a stronger presence of the repetition.

Spectral analysis techniques, such as described in WO 2007/105,150, generally represent an improvement over manual DNA pattern analysis techniques, which aim at identifying DNA patterns serving as biological markers related to important biological processes. Traditionally, automatic analyses are performed directly on strings of DNA sequences composed of the four characters A, T, C and G, which represent the four nucleotide bases. However, due to the tremendous length of DNA sequences (e.g., the length of the shortest human chromosome is 46.9Mb), the wide range of pattern spans associated with the limited character set, and the statistical nature of the problem, such an intuitive/manual approach is inefficient, if not impossible, for achieving the desired purpose.

Hence, an improved method for DNA sequence analysis would be advantageous and in particular a method allowing for increased flexibility, cost-effectiveness, or faster DNA sequence analysis would be advantageous.

SUMMARY OF THE INVENTION

Accordingly, the present invention preferably seeks to mitigate, alleviate or eliminate one or more of the above-identified deficiencies in the art and disadvantages singly or in any combination and solves at least the above mentioned problems e.g. by providing a method for nucleotide sequence analysis based on nucleotide spectrogram database. Such database may e.g. be a DNA database or a RNA database, well known to a person skilled in the art.

In an aspect a method for DNA sequence analysis is provided. The method comprises building a DNA spectrogram database based on a DNA database comprising a number of sequences of nucleotides, by calculating an energy spectral density value for each group of nucleotides comprised in the DNA database. The method further comprises inputting a DNA query sequence. Moreover, the method comprises calculating an energy spectral density value for the DNA query sequence, resulting in an energy spectral density query. The method further comprises calculating a difference between the energy spectral density query value and an energy spectral density value comprised in the DNA spectrogram database. Furthermore, the method comprises selecting a calculated difference, pertaining to a first group of nucleotides, being within a predetermined threshold value range ($\pm\Phi_\Lambda$).

In another aspect a use of the method in designing a test kit for diagnosing genetic diseases is provided.

In an aspect a device comprising a processor unit is provided. The processor unit is configured to build a DNA spectrogram database based on a DNA database comprising a number of sequences of nucleotides, by calculating an energy spectral density value for a group of nucleotides comprised in the DNA database. The processor unit is further configured to receive a DNA query sequence. Moreover, ther processor unit is configured to calculate an energy spectral density value for the DNA query sequence, resulting in an energy spectral density query. Furthermore, the processor unit is configured to calculate a difference between the energy spectral density query value and an energy spectral density value comprised in the DNA spectrogram database. The processor unit is further configured to select a difference being lower than a predetermined threshold value.

In yet another aspect a computer-readable medium having embodied thereon a computer program for processing by a processor is provided. The computer program comprises a first code segment for building a DNA spectrogram database based on a DNA database comprising a number of sequences of nucleotides, by calculating an energy spectral density value for a group of nucleotides comprised in the DNA database. The computer program further comprises a second code segment for inputting a DNA query sequence. Moreover, the computer program comprises a third code segment for calculating an energy spectral density value for the DNA query sequence, resulting in an energy spectral density query. Furthermore, the computer program comprises a fourth code segment for calculating a difference between the energy spectral density query value and an energy spectral density value comprised in the DNA spectrogram database. The computer program also comprises a fifth code segment for selecting a difference being lower than a predetermined threshold value.

The method may comprise the steps of building a DNA spectrogram database. The spectrogram database may be based on a DNA database comprising a number of sequences of nucleotides. This may be done by calculating an energy spectral density value for a group of nucleotides comprised in the DNA database. A DNA query sequence may be used as an input. The energy spectral density value for the DNA query sequence may be calculated, resulting in an energy spectral density query. Then, a difference between the energy spectral density query value and an energy spectral density value comprised in the DNA spectrogram database may be calculated. After this, a calculated difference, pertaining to a first group of nucleotides, being within a predetermined threshold value range ($\pm\Phi_\Delta$) may be selected.

The present invention according to some embodiments has the advantage over the prior art that it provides a possibility to compare sequences with large number of nucleotides. Moreover, the improved sequence comparison may also be performed faster than current solutions.

Other embodiments of the invention will be explained in further detail below.

BRIEF DESCRIPTION OF THE DRAWINGS

These and other aspects, features and advantages of which the invention is capable of will be apparent and elucidated from the following description of embodiments of the present invention, reference being made to the accompanying drawings, in which

Fig. 1    is a flowchart of a method according to an embodiment;
Fig. 2    is a flowchart of the building step of the method according to an embodiment; and
Fig. 3    is a block diagram of a device according to according to an embodiment.
Fig. 4    is a block diagram of a computer-readable medium according to an embodiment.

DETAILED DESCRIPTION OF EMBODIMENTS

Several embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the invention is only limited by the appended patent claims. Furthermore, the terminology used in the detailed description of the particular embodiments illustrated in the accompanying drawings is not intended to be limiting of the invention.

The following description focuses on embodiments of the present invention applicable to efficient searching of DNA Sequence in a DNA sequence database based on energy bands of DNA Spectrogram.

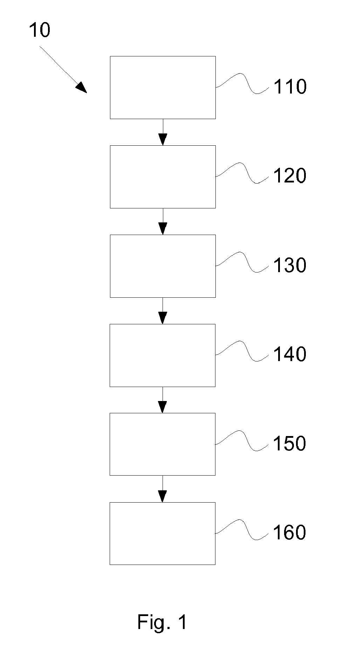

In an embodiment, according to Fig. 1, a method 10 for DNA sequence analysis is disclosed. The method comprises building 110 a DNA spectrogram database based on a DNA database comprising a number of sequences of nucleotides, by calculating an energy spectral density value for a group of nucleotides comprised in the DNA database. The method may further comprise inputting 120 a DNA query sequence. Moreover, the method comprises calculating 130 an energy spectral density value for the DNA query sequence, resulting in an energy spectral density query. Furthermore, the method may comprise calculating a difference 140 between the energy spectral density query value and an energy spectral density value comprised in the DNA spectrogram database. The method may also comprise selecting 150 a difference being lower than a predetermined threshold value.

The group of nucleotides, corresponding to the selected difference, may then be further processed using sequence alignment e.g. a BLAST algorithm. Accordingly, the method may further comprise performing 160 sequence alignment the nucleotides comprised in a selected group.

According to one embodiment, the DNA spectrogram database is an energy spectral density (ESD) database. The DNA spectrogram database may be a genomic DNA spectral database. The ESD describes how the energy (or variance) of a signal or a time series is distributed with frequency. If f(t) is a finite-energy (square integrable) signal, the spectral density $\Phi(\omega)$ of the signal is the square of the magnitude of the continuous Fourier transform of the signal. The energy is represented by the integral of the square of a signal.

As the signal is discrete with values $f_n$, over an infinite number of elements, we still have an energy spectral density:

$$\Phi(\omega) = \left| \frac{1}{\sqrt{2\pi}} \sum_{n=-\infty}^{\infty} f_n e^{-j\omega n} \right|^2 = \frac{F(\omega)F^*(\omega)}{2\pi}$$

where is the angular frequency ($2\pi$ times the cycle frequency) and $F(\omega)$ is the discrete-time Fourier transform of $f_n$, and $F^*(\omega)$ is its complex conjugate. The multiplicative factor of $1/2\pi$ is not absolute, but rather depends on the particular normalizing constants used in the definition of the various Fourier transforms.

According to one embodiment a set of color spectrums of the nucleotide segment, such as a DNA segment, is achived in a way well known to a person skilled in the art. Next, the periodicity of different color spectrums is calculated by the formula:

$$Periodicity = \frac{STFT\ Window\ Size}{Frequency}$$

Here, STFT Window Size is the window size calculated by Short Time Fourier Transform (STFT), well known to a person skilled in the art, and Frequency is the frequency of which a certain color spectrum is occuring when the different color spectrums are aligned. For a particluar STFT Window Size, Discrete Fourier Transforms (DFT) are combined in the color space, indicating a certain frequency. Then, the DFT values are squared and divided with the STFT Window Size to get the ESD.

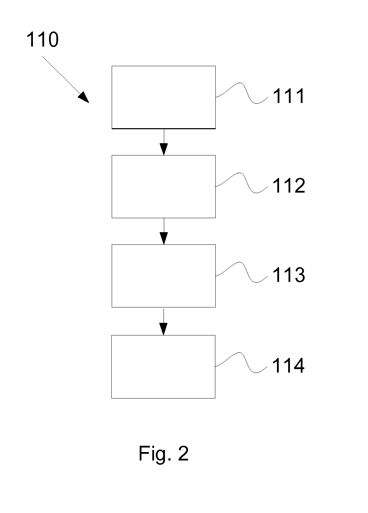

In an embodiment according to Fig. 2, the building 110 of a DNA spectrogram database is shown. First DNA spectrograms are pre-computed 111 for a large number of genome sequences. A large number of ESD are computed according to above for various lengths of sequences, comprised in a DNA sequence database, and various overlapping starting points. Such pre-computed ESD values may be used as part of the header information of the query sequence similar to a FASTA header, known in the art. The ESD values may differ for a range of nucleotide lengths, e.g. $\Phi_1, \Phi_2, ...,$ $\Phi_n$ for nucleotide lengths 256, 1024 ... , 8196 respectively. This may trigger the query and make another computation of ESD unnecessary. For example, in a certain color space, ESD computation may be derived by squaring DFT values and dividing them by the STFT Window Size.

The building 110 of the DNA spectrogram database may further comprise indexing 112 the pre-computed 111 DNA spectrograms in a structure based on phylogenetic distances. The building 110 of the DNA spectrogram database may further comprise assigning 113 a pointer to the spectrograms. Such pointer may be e.g. a reference to a local database, a URL to a web resource or a protected sequence. The spectrograms may then be stored 114.

In an embodiment, an ESD database may be used in such a way as to provide a fast baseline of probable candidates of sequences from the DNA sequence database, wherein the candidates may be related to the query sequence based on the ESD. Accordingly, the candidates having a similar ESD value to the ESD value of the query sequence may rapidly be identified for further processing. This is due to the fact that the method identifies sequences having similar ESD values to the ESD value of the query sequence. Accordingly, sequences having ESD values within $\pm\Phi_\Delta$, may be selected for subsequent processing.

The ESD database also gives the possibility to identify mutations in the DNA sequence. If the specific DNA sequence location e.g. already is known, the energy spectral density ($\Phi_{Ref}$) of the "healthy / valid" sequence is computed. In order to check for any mutation at that location in other DNA sequences, instead of comparing the sequence per nucleotide, in accordance with current solutions, the "energy spectral density" may be computed directly and changes in value of the "energy spectral density ($\Phi_{sam}$)" may be checked for. If $\Phi_{Ref} \neq \Phi_{sam}$, then there is a mutation, and whether it is fatal or not needs to be compared in depth using the existing search tools like BLAST.

In another embodiment the method comprises comparing "entire" chromosome or genomic sequence against the database of stored sequences without any huge penalty of comparing every nucleotide for producing search results, as the comparison is based on the "energy spectral density".

According to one embodiment, the sequence alignment 160 is local alignment, such as alignment of short sequences or alignment of shot-gun sequencing results.

According to another embodiment, the sequence alignment 160 is global alignment, such as alignment of multiple sequences all at once or alignment of two or more genomes.

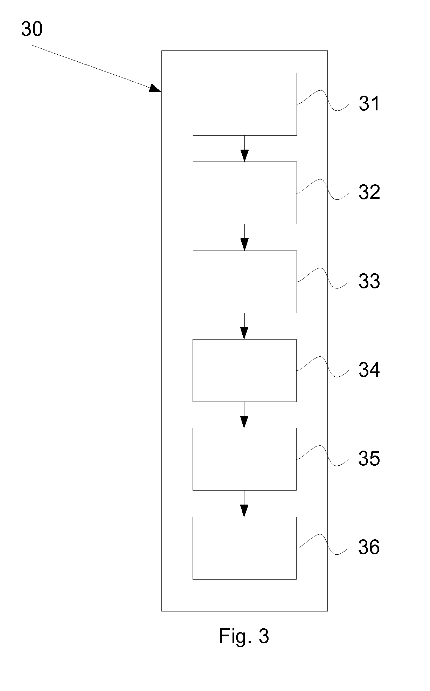

In an embodiment, according to Fig. 3, a device 30 is provided. The device comprises a processor unit configured to build 31 a DNA spectrogram database based on a DNA database comprising a number of sequences of nucleotides, by calculating an energy spectral density value for a group of nucleotides comprised in the DNA database. The processor unit is further configured to receive 32 a DNA query sequence. Moreover, the processor is configured to calculate 33 an energy spectral density value for the DNA query sequence, resulting in an energy spectral density query. Furthermore, the processor unit is configured to calculate 34 a difference 140 between the energy spectral density query value and an energy spectral density value comprised in the DNA spectrogram database. The processor unit is further configured to select 35 a difference being lower than a predetermined threshold value.

In an embodiment the processor unit is further configured to perform 36 sequence alignment the nucleotides comprised in a selected group.

In an embodiment the processor unit is configured to perform any one of the steps of the method according to some embodiments.

According to another embodiment, any of the abovementioned method may be used for designing test kits for diagnosing genetic diseases.

In one embodiment, a clinical genetics program is disclosed, the program comprising means to provide fast access to similar genomes of patients with similar disease conditions or provide fast access to similar patients with similar therapy response. The program may also comprise information from pharmacological databases for therapy response and associated genes with this therapy response as well as storage of genomic sequencing (like PACS for medical image).

According to one embodiment, genome-sequencing equipment is disclosed; the equipment needs to assemble full genomes.

Applications and use of the above-described method according to the invention are various and include exemplary fields such as clinical genetics or clinical genomics.

In an embodiment the device is comprised in a system adapted to operate and/or perform the method according to some embodiments. The system may be a medical workstation or medical system, such as a Computed Tomography (CT) system, Magnetic Resonance Imaging (MRI) System or Ultrasound Imaging (US) system.

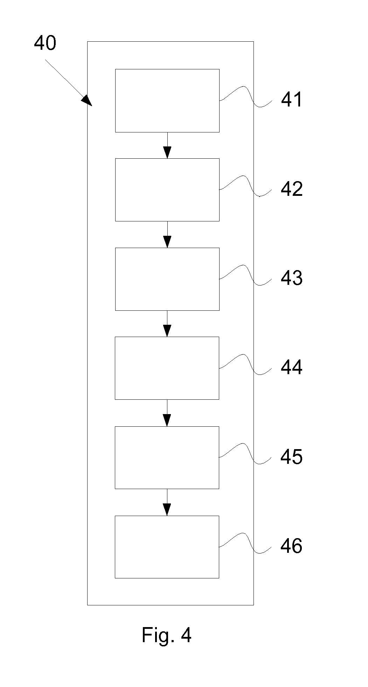

In an embodiment, according to Fig. 4, a computer-readable medium is provided having embodied thereon a computer program for processing by a processor. The computer program comprises a first code segment 41 for building 110 a DNA spectrogram database based on a DNA database comprising a number of sequences of nucleotides, by calculating an energy spectral density value for a group of nucleotides comprised in the DNA database; a second code segment 42 for inputting 120 a DNA query sequence; a third code segment 43 for calculating 130 an energy spectral density value for the DNA query sequence, resulting in an energy spectral density query; a fourth code segment 44 for calculating a difference 140 between the energy spectral density query value and an energy spectral density value comprised in the DNA spectrogram database; and a fifth code segment 45 for selecting 150 a difference being lower than a predetermined threshold value.

In an embodiment the computer program further comprise a sixth code segment for performing 46 sequence alignment the nucleotides comprised in a selected group.

In an embodiment the computer program comprises code segments arranged, when run by an apparatus having computer-processing properties, for performing any one of the method steps defined in some embodiments.

The invention may be implemented in any suitable form including hardware, software, firmware or any combination of these. However, preferably, the invention is implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. The terms DNA sequence and DNA spectrogram database, as represented in the claims, may be any nucleotide sequence, or nucleotide spectrogram database, which is easily understood by a person skilled in the art. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

**Claims**

**1.** A method (10) for DNA sequence analysis, comprising:

building (110) a DNA spectrogram database based on a DNA database comprising a number of sequences of nucleotides, by calculating an energy spectral density value for each group of nucleotides comprised in said DNA database,
inputting (120) a DNA query sequence;
calculating (130) an energy spectral density value for said DNA query sequence, resulting in an energy spectral density query;
calculating a difference (140) between said energy spectral density query value and an energy spectral density value comprised in the DNA spectrogram database; and
selecting (150) a calculated difference, pertaining to a first group of nucleotides, being within a predetermined threshold value range ( $\pm \Phi_\Delta$ ).

**2.** The method according to claim 1, further comprising performing sequence alignment (160) on said first group of nucleotides from the DNA spectrogram database.

**3.** The method according to claim 1, wherein said DNA spectrogram database is a genomic energy spectral density database.

**4.** The method according to claim 3, wherein said sequence alignment (160) is local alignment.

**5.** The method according to claim 3, wherein said sequence alignment (160) is global alignment.

**6.** Use of the method according to claim 1 in designing a test kit for diagnosing genetic diseases.

**7.** A device comprising a processor unit configured to:

build (31) a DNA spectrogram database based on a DNA database comprising a number of sequences of nucleotides, by calculating an energy spectral density value for a group of nucleotides comprised in the DNA database;
receive (32) a DNA query sequence;
calculate (33) an energy spectral density value for the DNA query sequence, resulting in an energy spectral density query;
calculate (34) a difference between the energy spectral density query value and an energy spectral density value comprised in the DNA spectrogram database; and
select (35) a difference being lower than a predetermined threshold value.

**8.** A computer-readable medium having embodied thereon a computer program for processing by a processor, said computer program comprising:

a first code segment (41) for building a DNA spectrogram database based on a DNA database comprising a number of sequences of nucleotides, by calculating an energy spectral density value for a group of nucleotides comprised in the DNA database;
a second code segment (42) for inputting a DNA query sequence;
a third code segment (43) for calculating an energy spectral density value for the DNA query sequence, resulting in an energy spectral density query;
a fourth code segment (44) for calculating a difference between the energy spectral density query value and an energy spectral density value comprised in the DNA spectrogram database; and
a fifth code segment (45) for selecting a difference being lower than a predetermined threshold value.

10

110

120

130

140

150

160

Fig. 1

110

111

112

113

114

Fig. 2

Fig. 3

Fig. 4

Europäisches
Patentamt

European
Patent Office

Office européen
des brevets

# EUROPEAN SEARCH REPORT

Application Number

EP 08 16 9327

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AGHILI S A ET AL: "Filtration of string proximity search via transformation" PROCEEDINGS, THIRD IEEE SYMPOSIUM ON BIOINFORMATICS AND BIOENGINEERING (BIBE 2003), PISCATAWAY, NJ, USA, 10 March 2003 (2003-03-10), pages 149-157, XP010637090 ISBN: 978-0-7695-1907-4 * abstract * * page 151, left-hand column, paragraph 2 - page 153, right-hand column, paragraph 2 * * page 156, right-hand column, paragraph 2 - page 157, left-hand column, paragraph 1 * * page 159, left-hand column, paragraph 1 - right-hand column, paragraph 2 * | 1-8 | INV. G06F19/00 |
| X | TUAN D PHAM ET AL: "LPC-VQ based Hidden Markov Models for Similarity Searching in DNA Sequences" IEEE INTERNATIONAL CONFERENCE ON SYSTEMS, MAN AND CYBERNETICS (ICSMC '06), 1 October 2006 (2006-10-01), pages 1654-1659, XP031117176 ISBN: 978-1-4244-0099-7 * abstract * * page 1654, left-hand column, paragraph 1 - right-hand column, paragraph 3 * * page 1655, left-hand column, paragraph 3 - paragraph 4 * * page 1656, left-hand column, paragraph 1 - right-hand column, paragraph 1 * | 1-8 | TECHNICAL FIELDS SEARCHED (IPC) G06F G06K |

-/--

The present search report has been drawn up for all claims

| Place of search | Date of completion of the search | Examiner |
|---|---|---|
| The Hague | 23 January 2009 | Türkeli, Yasemin |

EPO FORM 1503 03.82 (P04C01)

Europäisches
Patentamt

European
Patent Office

Office européen
des brevets

# EUROPEAN SEARCH REPORT

Application Number

EP 08 16 9327

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NAGARAJAN VIJAYARAJ ET AL: "A Fourier transformation based method to mine peptide space for antimicrobial activity" BMC BIOINFORMATICS, vol. 7, no. Suppl. 2, September 2006 (2006-09), pages 1-8, XP002511632 ISSN: 1471-2105 * abstract; figure 1 * * page 2, right-hand column, paragraph 3 - page 5, left-hand column, paragraph 2 * * page 6, left-hand column, paragraph 3 - page 7, left-hand column, paragraph 4 * | 1-8 | |
| A | MATTEO RÃ CR ET AL: "Signal Processing in Comparative Genomics" APPLICATIONS OF FUZZY SETS THEORY; [LECTURE NOTES IN COMPUTER SCIENCE], SPRINGER, BERLIN, HEIDELBERG, vol. 4578, 7 July 2007 (2007-07-07), pages 544-550, XP019064613 ISBN: 978-3-540-73399-7 * abstract * * page 546, paragraph 1 - page 548, paragraph 2 * | 1-8 | |
| A | WO 2006/124760 A (PANVIA FUTURE TECHNOLOGIES INC [US]; SELLY ROGER [US]) 23 November 2006 (2006-11-23) * abstract; figures 1-5 * * page 1, paragraph 2 * * page 10, paragraph 4 - page 11, paragraph 3 * * page 13, paragraph 2 * * page 15, paragraph 2 - page 22, paragraph 3 * | 1-8 | TECHNICAL FIELDS SEARCHED (IPC) |

-/--

The present search report has been drawn up for all claims

| Place of search | Date of completion of the search | Examiner |
|---|---|---|
| The Hague | 23 January 2009 | Türkeli, Yasemin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 08 16 9327

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | WO 2007/105150 A (KONINKL PHILIPS ELECTRONICS NV [NL]; CHEUNG YEE HIM [US]; DIMITROVA NE) 20 September 2007 (2007-09-20) * abstract * * page 3, paragraph 2 - page 8, paragraph 1 * ----- | 1-8 | |

TECHNICAL FIELDS
SEARCHED (IPC)

The present search report has been drawn up for all claims

| Place of search | Date of completion of the search | Examiner |
|---|---|---|
| The Hague | 23 January 2009 | Türkeli, Yasemin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**                    EP 08 16 9327

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2009

| Patent document cited in search report | | Publication date | Patent family member(s) | | Publication date |
|---|---|---|---|---|---|
| WO 2006124760 | A | 23-11-2006 | EP | 1886226 A2 | 13-02-2008 |
| WO 2007105150 | A | 20-09-2007 | EP | 1999663 A2 | 10-12-2008 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007105150 A